# EUROPEAN PATENT APPLICATION

(11) **EP 1 898 328 A2**
(43) Date of publication of application: **12.03.2008**
(21) Application number: 07252173.5
(22) Date of filing: 25.05.2007
(51) Int. Cl.: G06F 19/00

(54) **An interface between clinical and research information systems**

(30) Priority: 01.09.2006 US 824336 P; 30.04.2007 US 741823
(71) Applicant: Siemens Medical Solutions USA, Inc., Malvern, PA 19355-1406 (US); Siemens Aktiengesellschaft, 80333 Munich (DE)
(72) Inventor: Zahlmann, Gudrun Wirkner, 92318 Neumarkt (DE); Brandon, Paul, Sanatoga Pennsylvania 19464 (US); Wronka, Andrew, New Jersey 07758 (US); Schmidt, Markus, 94025 Nuernberg (DE)
(74) Representative: Clarke, Alison Clare

(57) **Abstract**

A system provides a clinical trial or research process with access to, and use of information employed in patient care by facilitating access to information via a portal (e.g., a Web compatible portal) and providing automated transfer of data collected during patient care workflows for storage in clinical research information systems. An interface system enables bidirectional exchange of data between a hospital clinical information system and a clinical trial or research information system. The interface system employs at least one repository including information associating a clinical trial with patient identifiers, healthcare worker identifiers, an indicator of individual status of a patient in the clinical trial and an identifier of a site involved in the clinical trial. An interface processor provides medical information of a particular patient, acquired using a hospital clinical information system, to a clinical trial or research storage repository by using information derived from the at least one repository for automatically determining, the particular patient is enrolled in a clinical trial and the particular patient participation in the clinical trial is still current.

## Description

This is a non-provisional application of provisional application serial No. 60/824,336 by P. Brandon et al. filed September 1, 2006.

### Field of the Invention

This invention concerns an interface system enabling bidirectional exchange of data between a hospital clinical information system and a clinical trial or research information system.

### Background of the Invention

Clinical research typically involves use of a fragmented environment involving different computer information systems for specific tasks. It is desirable to provide integrated data access and work flow in such an environment. Especially, in supporting clinical trials that have to follow strict protocols, seamless integration of electronically available data from different source systems is of substantial benefit. In existing clinical research systems, researchers typically fill in paper forms with clinical data describing the state of a patient in a clinical trial. Often this data is electronically available, e.g., a hematocrit value is available from a laboratory system. Further, existing systems lack integration between a laboratory system and a clinical research environment which necessitates replicated data entry in a trial and this is a burdensome error prone process. In existing systems a user is obliged to search for clinical trial related data manually in different electronic source systems or even to search paper medical records.

Complex clinical information systems are managed and supported by dedicated staff and are difficult to adjust to new requirements. This impairs operation in an innovation focussed, clinical trial and research environment typically requiring a flexible approach and which may involve only a few patients, (e.g., ten) for a short time. Also, clinical information systems include data sources offering widely varying degrees of data availability, including, for example, (i) largely integrated database systems or data warehouses and (ii) small systems with a limited number of data items per system. Integration technologies exist for both alternatives. However, there is a lack of a comprehensive integrated technical system that integrates clinical information systems and clinical research systems.

Computer system integration techniques have existed for a long time. Enterprise Application Integration (EAI) is employed for small numbers of systems involving exchange of limited numbers of messages between systems to integrate different application functions. Integration involves translating one message format into another to be compatible with another system as required. Messages are sent on request without formal data integration into a centralised data repository. Another integration system is an Extract Transform Link (ETL) system that integrates different existing centralised data repositories or data warehouses by making data interchangeable at the database level. Hospital information system environments may employ both (and other) integration techniques. Nevertheless dedicated computer staff are still required to implement the integration. This deters researchers from using such systems since they are complex and time consuming to integrate. In addition, existing integration systems fail to support work flow operation. A system according to invention principles addresses these deficiencies and associated problems. Specifically, the inventors have advantageously recognized that clinical researchers are also usually clinicians that spend the majority of their time in routine clinical care. In between routine care activities or after normal work they do research. Therefore it is desirable to provide an easy to use trial information system integrated with clinical systems employed in clinician routine and enable integrated workflow management.

### Summary of the Invention

A system provides a clinical trial or research process with access to, and use of information employed in patient care by facilitating access to information via a portal (e.g., a Web compatible portal) and providing automated transfer of data collected during patient care workflows for storage in clinical research information systems. An interface system enables bidirectional exchange of data between a hospital clinical information system and a clinical trial or research information system. The interface system employs at least one repository including information associating a clinical trial with patient identifiers, healthcare worker identifiers, an indicator of individual status of a patient in the clinical trial and an identifier of a site involved in the clinical trial. An interface processor provides medical information of a particular patient, acquired using a hospital clinical information system, to a clinical trial or research storage repository by using information derived from the at least one repository for automatically determining, the particular patient is enrolled in a clinical trial and the particular patient participation in the clinical trial is still current.

### Brief Description of the Drawing

Figure 1 shows an interface system enabling bidirectional exchange of data between a hospital clinical information system and a clinical trial or research information system, according to invention principles.
Figure 2 shows a further interface system enabling bidirectional exchange of data between a hospital clinical information system and a clinical trial or research information system, according to invention principles.
Figure 3 shows a table including data indicating clinical trial characteristics, according to invention principles.
Figure 4 shows a table including data indicating patient identifiers and patient characteristics, according to invention principles.
Figure 5 shows a table including data indicating clinical trial worker characteristics, according to invention principles.

### Detailed Description of the Invention

A processor, as used herein, operates under the control of an executable application to (a) receive information from an input information device, (b) process the information by manipulating, analyzing, modifying, converting and/or transmitting the information, and/or (c) route the information to an output information device. A processor may use, or comprise the capabilities of, a controller or microprocessor, for example. The processor may operate with a display processor or generator. A display processor or generator is a known element for generating signals representing display images or portions thereof. A processor and a display processor comprise any combination of, hardware, firmware, and/or software.

An executable application, as used herein, comprises code or machine readable instructions for conditioning a processor to implement predetermined functions, such as those of an operating system, a context acquisition system or other information processing system, for example, in response to user command or input. An executable procedure is a segment of code or machine readable instruction, subroutine, or other distinct section of code or portion of an executable application for performing one or more particular processes. These processes may include receiving input data and/or parameters, performing operations on received input data and/or performing functions in response to received input parameters, and providing resulting output data and/or parameters.

A user interface (UI), as used herein, comprises one or more display images, generated by a display processor and enabling user interaction with a processor or other device and associated data acquisition and processing functions. The UI also includes an executable procedure or executable application. The executable procedure or executable application conditions the display processor to generate signals representing the UI display images. These signals are supplied to a display device which displays the image for viewing by the user. The executable procedure or executable application further receives signals from user input devices, such as a keyboard, mouse, light pen, touch screen or any other means allowing a user to provide data to a processor. The processor, under control of an executable procedure or executable application manipulates the UI display images in response to the signals received from the input devices. In this way, the user interacts with the display image using the input devices, enabling user interaction with the processor or other device. The functions and process steps herein may be performed automatically or wholly or partially in response to user command. An activity (including a step) performed automatically is performed in response to executable instruction or device operation without user direct initiation of the activity. Workflow comprises a sequence of tasks performed by a device or worker or both. An object or data object comprises a grouping of data, executable instructions or a combination of both or an executable procedure. A document or record comprises a compilation of data in electronic or paper form.

A workflow processor, as used herein, processes data to determine tasks to add to a task list, remove from a task list or modifies tasks incorporated on, or for incorporation on, a task list. A task list is a list of tasks for performance by a worker or device or a combination of both. A workflow processor may or may not employ a workflow engine. A workflow engine, as used herein, is a processor executing in response to predetermined process definitions that implement processes responsive to events and event associated data. The workflow engine implements processes in sequence and/or concurrently, responsive to event associated data to determine tasks for performance by a device and or worker and for updating task lists of a device and a worker to include determined tasks. A process definition is definable by a user and comprises a sequence of process steps including one or more, of start, wait, decision and task allocation steps for performance by a device and or worker, for example. An event is an occurrence affecting operation of a process implemented using a process definition.

A Workflow Management System is a software system that manages processes. It includes a process definition function that allows users to define a process that should be followed, an Event Monitor, which captures events from a Healthcare Information System and communicates the results to the Workflow Management System. A processor in the Management System tracks which processes are running, for which patients, and what step needs to be executed next, according to a process definition. The Management System includes a procedure for notifying clinicians of a task to be performed, through their worklists and a procedure for allocating and assigning tasks to specific users or specific teams.

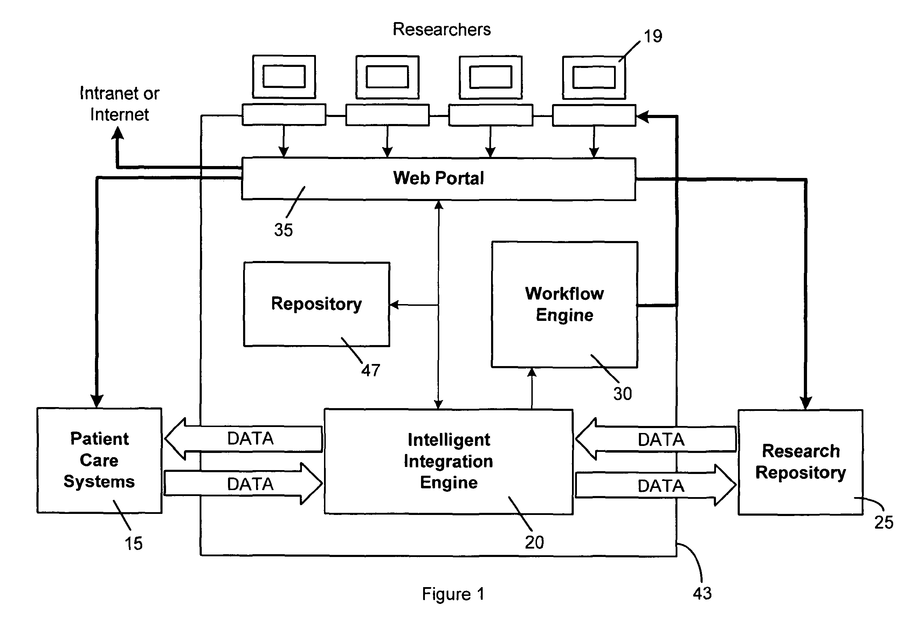

Figure 1 shows interface system 10 including unit 43 enabling bidirectional exchange of data between a hospital clinical information system 15 and a clinical trial or research information system or repository 25 (e.g. a research repository, an Electronic Data Capture system or a CDMS (Chemical Data Management System) system). Repository 47 (comprising one or more databases) includes information associating a clinical trial with patient identifiers, healthcare worker identifiers, an indicator of individual status of a patient in the clinical trial and an identifier of a site involved in the clinical trial. Interface processor (integration engine) 20 provides medical information of a particular patient, acquired using a hospital clinical information (patient care) system 15, to a clinical trial or research storage repository 25. Interface processor 20 does this by using information derived from repository 47 for automatically determining, that the particular patient is enrolled in a clinical trial and the particular patient participation in the clinical trial is still current.

System 10 provides researchers using workstations 19 with improved access to information used in a patient care process for use in a clinical research process by facilitating access to information via portal 35. System 10 provides automated transfer of data collected during patient care workflows (conforming to standard care protocols) for storage in clinical research systems. System 10 includes in one embodiment, Web Portal 35, an interface processor 20 and Workflow Engine 30. These units are integrated into system 10 and support user workflows for both clinical research and patient care and enable electronic transfer of data between patient care system (comprises systems in clinical routine and existing data sources that can be leveraged - these systems may use a common Universal ID (UID)) 15 and clinical research repository and systems 25. System 10 includes a single sign on function enabling a user to login once to one application and gain access to multiple different executable applications. A context processor in system 10 seamlessly provides context data (e.g., a patient identifier, user identifier, task identifier etc.) between multiple different executable applications. Further, interface processor 20 is able to access clinical systems used in normal patient care and clinical trial research and provides display image views of clinical and research data in a researcher friendly manner. The integrated system enables a user to select data processing functions and initiate data transfer and other tasks in a quick and easy manner without programmer involvement.

Web Portal 35 provides a user with a single sign on environment to a comprehensive array of executable applications used by a clinical researcher including clinical information systems employed in routine patient care as well as clinical research systems. In addition, Web Portal 35 provides storage of context data for each patient. For example, Web Portal 35 tracks patient and worker identifiers, for example, used by different systems including a Healthcare Information System (HIS), a laboratory system, a Pharmacy system, a Radiology Information System and an Electronic Data Capture system along with a patient first name, last name, date of birth and a clinical trial the patient is enrolled in. A configuration processor (not shown) in unit 43 enables a user such as a researcher to determine which data elements are stored to support clinical trial requirements

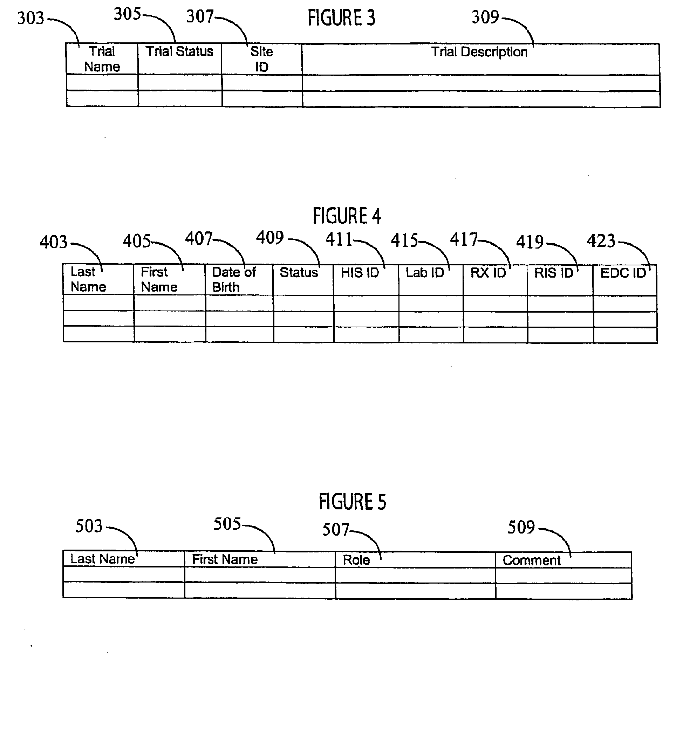

Figure 3 shows a table including data indicating clinical trial characteristics employed by interface processor 20 (and portal 35). Stored data elements may include, for example, data indicating a list of clinical trials with associated corresponding lists of patients identifying patients enrolled in an individual trial and a list of research staff involved in the individual trial. Stored data elements also include clinical trial name 303, trial description 309, trial status 305 and site identifier 307.

Web Portal 35 provides a user interface allowing user manual data entry and viewing of patient identifiers and other data acquired from each of the systems as well as any additional information. Web Portal 35 may also import patient identifiers and other patient information. Web portal 35 further retains data indicating, status of a patient in a clinical trial, indicating if the patient is actively participating in the trial or if the patient is no longer participating in the trial, as well as a list of research staff involved with a trial including information indicating first and last name of staff members and their role in the trial, for example. Figure 4 shows a table employed by portal 35 and processor 20, including data indicating patient identifiers and patient characteristics. Specifically, the table includes patient last and first name 403, 405, date of birth 407, patient status 409 and patient identifiers employed by different systems including an HIS identifier 411, a laboratory information system identifier 415, a pharmacy information system identifier 417, a radiology information system identifier 419 and an electronic data capture system identifier 423. Figure 5 shows a table including data indicating clinical trial worker characteristics. The table includes worker last and first name 503, 505, worker role 507 and worker comments 509 and can support additional identifiers and data as required. The Web Portal also records audit messages related to changes in setup of user account configuration parameters (e.g., enabling/disabling access privileges).

A context aware sign-on capability employed by Web Portal 35 uses stored patient identifiers and other information. Single sign-on function capabilities of portal 35 include an executable script implementing sign-on steps, alternatively portal 35 may use an application programming interface, to ensure a user arrives at a point in the application at which desired patient specific data is displayed. Portal 35 incorporates a patient aware sign-on function implemented using an available scripting application (e.g., WinBatch® available from Wilson WindoWare) to emulate keystrokes of a user and provide information from a patient index to select menu options related to display of patient information, for example. An application system being accessed also provides functions that manage user access rights. A search and query function enables a user to generate a query that uses integration engine 20 to obtain a particular system identifier used by a particular patient. The system uses a map in unit 20 that mutually associates patient (and other) identifiers of different systems (and the system names) enabling a user to determine an identifier employed by a computer system from a corresponding identifier employed by a different system. In operation, a transaction message is received by integration engine 20 from patient care system 15. The message includes an HIS identifier. Integration engine 20 sends an SQL compatible query or executes a stored procedure querying a patient index (a list of patients and associated patient identifiers) to request an identifier used for a particular patient by a research repository. Patient index related data is stored in a dedicated database in encrypted form in unit 43.

Web portal 35 provides a user with a single sign on environment to clinical and research information systems and advantageously maintains a list of login/password and other information needed to support automated sign-on to various systems. Portal 35 also provides seamless context data exchange between applications enabling a researcher to sign-on to a system and automatically convey to the system an identifier of a patient a researcher is concerned with. This automatic patient awareness capability ensures that execution of both healthcare provider and clinical trial systems is initiated with a focus on the same patient. Web portal 35 also supports secure login into different systems based on predetermined user role configuration data, time limited user associated account data, Internet Security protocols and password protection or other comparable functions and indicates to a user what data is available for access by a particular user with particular security privileges.

Interface processor 20 incorporates known standard Enterprise Application Integration and Extract, Transform and Load capabilities to pull or receive clinical trial related data, reformat data and to send or push the data into one or more destination systems. Processor 20 stores metadata describing individual acquired data elements including a semantic definition of a data element, a data type of a data element and associated allowed data element value ranges. Processor 20 in conjunction with web portal 35 and associated workstations 19 provides a user with a view of information derived from both patient care system 15 and clinical research repository and systems 25. Data is acquired and collated from units 15 and 25 using data (metadata) determining the meaning of the data in each system and presented by portal 35 via a workstation 19 in graphical form in one or more display images. The display images also provide a user with an ability to view the metadata for each data item. Interface processor 20 supports displaying information derived from a particular system in multiple views based upon the type of data acquired. For example, one view may display patient demographic data elements (such as age, gender, height, weight, address, etc.) maintained on the system and another view may show results of laboratory tests.

A display image provided by portal 35 on workstation 19 enables a user to choose data relevant to particular clinical trial research to be acquired from a data source system (e.g., an electronic laboratory information system) or to be sent to a destination system (e.g., an Electronic Data Capture system). A user employs a display image presented on workstation 19 to select a data element to be acquired from a source system and to select a corresponding data element to be sent to a destination system. Interface processor 20 suggests a link (association) between a source system (e.g., a Healthcare Information System (HIS)) 15 data element and a destination system 25 data element in response to stored metadata definitions of the data elements in each corresponding system. The data element link indicates that processor 20 is to communicate a data element from the source system to the destination system in response to occurrence of an event that adds to, or modifies data in system 10 or in response to access to system 10 data. Further, a portion of the data received by interface processor 20 from system 15 is communicated to clinical research repository 25. A user configures interface processor 20 using a configuration processor (not shown) in unit 43 to determine data elements that are to be permanently deleted, which data elements are to be forwarded unaltered and which data elements are to be reformatted prior to forwarding during the exchange of data between units 15 and 25. HIPAA compliant information identifying data accessing, editing and communication activities performed by interface processor 20 is collated and stored by an audit processor in unit 20. The stored data includes, a user identifier, patient identifier, source system identifier, destination system identifier, transaction message identifier, data and time of transaction and other data.

Interface processor 20 queries a context information map in Web Portal 35 that associates patient and other identifiers used by different systems to obtain a corresponding patient identifier used by destination system 25 to replace a patient identifier used in the source system 15 data. Processor 20 filters acquired data and forwards data to destination (e.g., clinical trial) system 25 that is related to a patient that has an active status in destination system 25 and excludes data concerning patients having an inactive status in system 25. For example, source medical data is received from source system 15 (e.g., an HIS system) for patient 12345678. Processor 20 queries repository 47 to obtain a corresponding identifier for a patient used by an Electronic Data Capture system. The query returns no value or a value indicating that the patient is not part of an active trial. Processor 20 discards the source data and does not forward it to destination system 25. Interface processor 20 transforms data received from source system 15 into a normalized data format and maps data in this normalized format to a format required by destination system 25. This mapping system advantageously enables re-use of mapping information for mapping from a source system to a common normalized format together with mapping information from the common normalized format to a destination system specific format. The mapping facilitates configuration of data exchange between system 15 and 25 and allows easy set-up of new data exchange interfaces.

In response to configuration of interface processor 20, data is communicated to destination research system 25 (e.g., an Electronic Data Capture system or Clinical Data Management system) from clinical systems 15 (e.g. HIS system, Laboratory information system, etc.) for patients identified in context information in Web Portal 35. Patient related medical data is also communicated from clinical and Research Systems 25 to update a patient medical record in clinical system 15. Interface processor 20 bidirectionally exchanges data between systems 15 and 25 and also provides a display of collated patient data in an image window that includes information used by various systems involved in clinical trial research based upon the meaning and context of the data in each system. A researcher logs into web portal 35 to view the collated information on workstation 19 provided by interface processor 20 using an Enterprise Application Integration (EAI) or Extract Transform Link (ETL) system, for example. A user selects via portal 35 and workstation 19, research relevant data available from data source system (e.g., an electronic laboratory system) 15 including data definitions, data types, value ranges and by clicking on the relevant items that are needed in clinical trial research system 25.

In one embodiment, interface processor 20 automatically employs data definitions and other metadata (data type, patient and other identifiers, diagnostic codes, medication codes, patient demographic data) provided by research system 25 to determine medical data in system 15 to be automatically communicated to system 25. Interface processor 20 supports forwarding data from clinical system 15 to research system 25 for particular patients selected via web portal 35 during system configuration or installation. In another embodiment, a user employs workstation 19 and web portal 35 to review data assembled and collated in system 15 using the data definitions and other metadata to select particular medical data items in system 15 to be communicated to system 25. Interface processor 20 communicates with systems recently integrated into system 10 (e.g., in unit 15 or 25) by identifying, the integration systems (e.g., ETL, EAI) employed, available data items and data messaging structure and formats, for example. Workflow engine 30 supports seamless information system integration for use by a clinician or researcher that readily supports addition of new clinical trials and configuring interface processor 20 for different tasks either in clinical routine patient care or in research.

Workflow engine 30 generates data messages and image data for display that guides a researcher to follow particular task activities to ensure proper Standard Operating Procedure (SOP) steps are observed in a proper order. Integration processor 20 initiates workflows that notify a researcher that new data has been processed and that a particular manual step (such as, manual review of received data) needs to be performed, for example. A workflow task sequence may await completion of a manual review (or other) step before proceeding to enable a next step in a SOP. Further, workflow engine 30 is configurable by a user via work station 19 and portal 35 to manage workflows of different worker roles involved in clinical trial research for a particular clinical trial. Engine 30 configures workflows in response to a predetermined clinical trial protocol. This facilitates ensuring that the proper Standard Operating Procedure (SOP) steps are observed in a proper order and that requirements of a clinical trial protocol are met. Interface processor 20 also generates alert messages for communication to workers occupying trial coordinator or other roles to investigate if a step in the workflow is not completed within a specified period of time, for example. In addition, interface processor 20 initiates a workflow that notifies a researcher that new data has been processed and that a particular manual step (such as, manual review of the received data) needs to be performed. For example, a transaction message is received from a laboratory information system 15 for patient 123 in trial ABC. Integration processor 20 processes the transaction message and sends predetermined data elements to an EDC system 25 and initiates a workflow that notifies a trial investigator responsible for a clinical trial to validate data that has been communicated to the EDC system. The workflow waits for completion of this step before proceeding to enable a next step in the SOP.

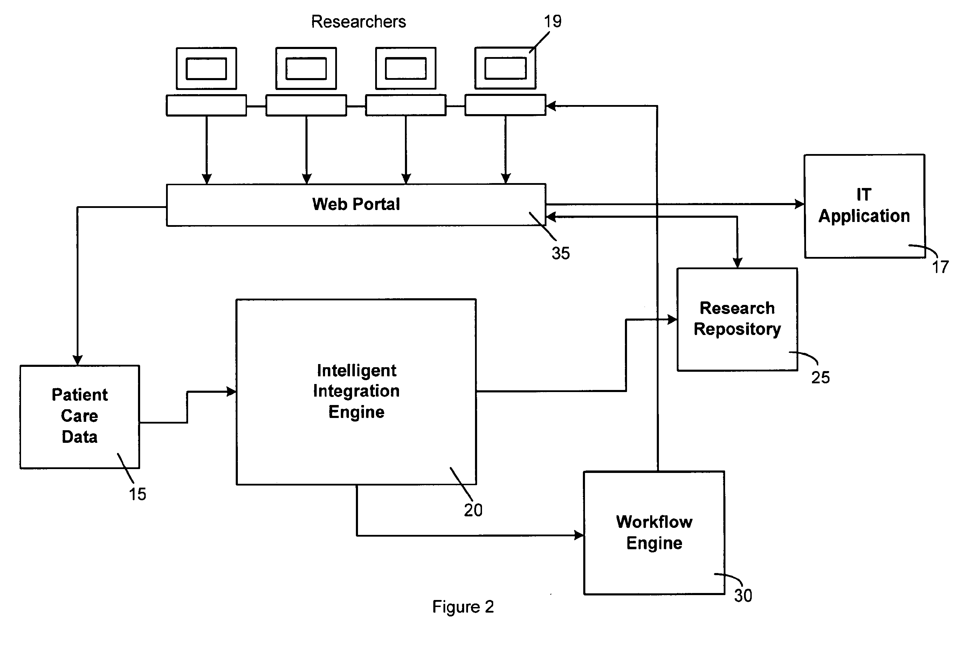

Figure 2 shows a further interface system enabling bidirectional exchange of data between a hospital clinical information system 15 and a clinical trial or research information system 25. A repository in unit 20 (comprising one or more databases) includes information associating a clinical trial with patient identifiers, healthcare worker identifiers, an indicator of individual status of a patient in the clinical trial and an identifier of a site involved in the clinical trial. Interface processor 20 automatically acquires patient medical information of a particular patient from a clinical trial site for communication to hospital clinical information system 15. Interface processor (integration engine) 20 automatically communicates medical information of a particular patient, acquired using a hospital clinical information (patient care) system 15, to a clinical trial or research storage repository 25. Interface processor 20 does this by using information derived from the repository for automatically determining, that the particular patient is enrolled in a clinical trial and the particular patient participation in the clinical trial is still current. The information in the repository associates the clinical trial with data indicating type of patient clinical information used by the clinical trial and with an indicator of status of the clinical trial and interface processor 20 filters the medical information of the particular patient provided to the clinical trial or research storage repository to be compatible with the type of patient clinical information used by the clinical trial.

A context information repository in unit 20 mutually associates identifiers of a patient used by multiple different systems including at least two of, (a) a laboratory Information System, (b) a Hospital Information System, (c) a Radiology Information System, (d) a Pharmacy Information System and (e) an Electronic Data Acquisition System. Interface processor 20 uses the context information in automatically acquiring the medical information of the particular patient from system 15. A context information map associates patient identifiers used by different systems to obtain a corresponding patient identifier used by destination system 25 including the clinical trial or research storage repository. Interface processor 20 queries the context information map to determine a patient identifier compatible with destination system 25 for incorporation in the medical information of the particular patient. Interface processor 20 converts the medical information of the particular patient to an intermediate data format and converts the medical information in the intermediate format to be compatible with destination system 25. Interface processor 20 filters the medical information of the particular patient and communicates filtered medical information to clinical trial or research storage repository 25 related to a patient that has an active status in the clinical trial. A display processor in workstation 19 (or web portal 35) provides data representing a composite display image identifying the medical information of the particular patient, acquired using hospital clinical information system 15, together with information indicating type of patient clinical information used by the clinical trial and enabling a user to select data items of the medical information to be provided by interface processor 20 to the clinical trial or research storage repository. This is done in response to an automatic determination performed using information in the repository indicating the particular patient is enrolled in a clinical trial. Portal 35 interacts with executable application 17 supporting data processing functions for display of data on workstation 19.

Interface processor 20 stores metadata describing individual data elements of the medical information of the particular patient including at least two of, (a) a semantic definition of a data element, (b) a data type of a data element and (c) associated allowed data element value ranges. Processor 20 uses the metadata to automatically identify individual data elements of the medical information to communicate to the clinical trial or research storage repository 25 in response to a determination, the particular patient is enrolled in a clinical trial and the particular patient participation in the clinical trial is still current. Workflow processor 30 provides message data to prompt a worker with tasks to ensure the user selects data items of the medical information complying with requirements of the clinical trial and using a selection process complying with requirements of the clinical trial.

The systems of Figures 1 and 2 are not exclusive. Other systems, processes and menus may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention. Further, any of the functions provided in the systems of Figures 1 and 2 may be implemented in hardware, software or a combination of both and may reside on one or more processing devices located at any location of a network linking system elements or another linked network including another intra-net or the Internet.

## Claims

1. An interface system enabling bidirectional exchange of data between a patient care system and a clinical trial or research information system, comprising:
at least one repository including information associating a clinical trial with patient identifiers, healthcare worker identifiers, an indicator of individual status of a patient in said clinical trial and an identifier of a site involved in said clinical trial; and
an interface processor for providing medical information of a particular patient, acquired using a hospital clinical information system, to a clinical trial or research storage repository by using information derived from said at least one repository for automatically determining,
said particular patient is enrolled in a clinical trial and
said particular patient participation in said clinical trial is still current.

2. A system according to claim 1, wherein
said information in said at least one repository associates said clinical trial with data indicating type of patient clinical information used by said clinical trial and
said interface processor filters said medical information of said particular patient provided to said clinical trial or research storage repository to be compatible with said type of patient clinical information used by said clinical trial.

3. A system according to claim 1, including
a context information repository for mutually associating identifiers of a patient used by multiple different systems including at least two of, (a) a laboratory Information System, (b) a Hospital Information System, (c) a Radiology Information System, (d) a Pharmacy Information System and (e) an Electronic Data Acquisition System wherein
said interface processor uses said context information in automatically acquiring said medical information of said particular patient.

4. A system according to claim 1, wherein
said interface processor converts said medical information of said particular patient to an intermediate data format and converts said medical information in said intermediate format to be compatible with a destination system.

5. A system according to claim 1, including
a display processor for providing data representing a composite display image identifying said medical information of said particular patient together with information indicating type of patient clinical information used by said clinical trial and enabling a user to select data items of said medical information to be provided by said interface processor to said clinical trial or research storage repository.

6. A system according to claim 5, including
a workflow processor for providing message data to prompt a worker with tasks to ensure said user selects data items of said medical information complying with requirements of said clinical trial.

7. A system according to claim 5, including
a workflow processor for providing message data to prompt a worker with tasks to ensure said user selects data items of said medical information using a process compliant with requirements of said clinical trial.

8. A system according to claim 1, wherein
said at least one repository includes information associating a clinical trial with an indicator of status of said clinical trial.

9. A system according to claim 1, wherein
said interface processor automatically acquires patient medical information of a particular patient from a clinical trial site for communication to said hospital clinical information system.

10. A system according to claim 1, wherein
said interface processor automatically communicates said medical information of said particular patient to said clinical trial or research storage repository in response to a determination, said particular patient is enrolled in a clinical trial and said particular patient participation in said clinical trial is still current.

11. A system according to claim 1, wherein
said interface processor filters said medical information of said particular patient and communicates filtered medical information to said clinical trial or research storage repository related to a patient that has an active status in said clinical trial.

12. A system according to claim 1, including
a context information map associating patient identifiers used by different systems to obtain a corresponding patient identifier used by a destination system including said clinical trial or research storage repository and wherein
said interface processor queries said context information map to determine a patient identifier compatible with said destination system for incorporation in said medical information of said particular patient.

13. A system according to claim 1, including
said interface processor stores metadata describing individual data elements of said medical information of said particular patient including at least two of, (a) a semantic definition of a data element, (b) a data type of a data element and (c) associated allowed data element value ranges.

14. A system according to claim 13, wherein
said interface processor uses said metadata to automatically identify individual data elements of said medical information to communicate to said clinical trial or research storage repository in response to a determination, said particular patient is enrolled in a clinical trial and said particular patient participation in said clinical trial is still current.

15. A system according to claim 11, wherein
a clinical trial comprises a research project.

16. An interface system enabling bidirectional exchange of data between a patient care system and a clinical trial or research information system, comprising:
at least one repository including information associating a clinical trial with patient identifiers, healthcare worker identifiers, an indicator of individual status of a patient in said clinical trial and an identifier of a site involved in said clinical trial;
a display processor for providing data representing a composite display image identifying medical information of a particular patient, acquired using a hospital clinical information system, together with information indicating type of patient clinical information used by said clinical trial and enabling a user to select data items of said medical information to be provided to a clinical trial or research storage repository, in response to an automatic determination said particular patient is enrolled in a clinical trial, said automatic determination being performed using information in said at least one repository; and
an interface processor for providing said selected data items of said medical information to said clinical trial or research storage repository.

17. An interface system enabling bidirectional exchange of data between a hospital clinical information system and a clinical trial or research information system, comprising:
at least one repository including information associating a clinical trial with patient identifiers, healthcare worker identifiers, an indicator of individual status of a patient in said clinical trial and an identifier of a site involved in said clinical trial;
a display processor for providing data representing a composite display image identifying medical information of a particular patient, acquired using a hospital clinical information system, together with information indicating type of patient clinical information used by said clinical trial and enabling a user to select data items of said medical information to be provided to a clinical trial or research storage repository, in response to an automatic determination said particular patient is enrolled in a clinical trial, said automatic determination being performed using information in said at least one repository; and
an interface processor for providing said selected data items of said medical information to said clinical trial or research storage repository and for automatically acquiring patient medical information of a particular patient from a clinical trial site for communication to said hospital clinical information system.
